# EUROPÄISCHE PATENTSCHRIFT

(11) **EP 1 028 678 B1**
(45) Veröffentlichungstag und Bekanntmachung des Hinweises auf die Patenterteilung: **12.05.2004**
(21) Anmeldenummer: 98949850.6
(22) Anmeldetag: 30.10.1998
(51) Int. Cl.: A61F 7/00

(54) **WÄRMEDECKE ZUM WÄRMEISOLIERENDEN ABDECKEN EINES PATIENTEN**
THERMAL COVER
ENVELOPPE THERMIQUE

(30) Priorität: 08.11.1997 DE 29719907 U
(43) Veröffentlichungstag der Anmeldung: 23.08.2000
(73) Patentinhaber: ZEBA AG, 4025 Basel (CH)
(72) Erfinder: WELTE, Thomas, D-79576 Weil am Rhein (DE); DIETLIN, Silvie, F-68580 Seppois-le-Bas (FR); DÖRFELT, Kurt, G., CH-5012 Schönenwerd (CH)
(74) Vertreter: Lusuardi, Werther Giovanni, Dr.
(86) Internationale Anmeldenummer: PCT/CH1998/000459
(87) Internationale Veröffentlichungsnummer: WO 1999/023979

(56) Entgegenhaltungen:
- WO-A-96/12454
- DE-A- 3 902 233
- FR-A- 2 598 886
- US-A- 5 383 918

## Beschreibung

Die Erfindung betrifft eine Vorrichtung zum wärmeisolierenden Abdecken eines Patienten gemäss dem Oberbegriff des Anspruchs 1.

Aus der WO96/12454 ist eine gattungsgemässe Wärmedecke bekannt, welche durch teilweise Beschichtung ihrer Oberfläche mit Polyurethan an diesen Stellen luftundurchlässig gemacht ist.

Der Erfindung liegt das Problem zugrunde, eine Wärmedecke mit verbesserten Eigenschaften zu schaffen.

Der Erfindung löst die gestellte Aufgabe mit einer Vorrichtung, welche die Merkmale des Anspruchs 1 aufweist.

Endlosfasern werden erfindungsgemäß verwendet, um die Partikelabgabe für diese Materialschicht tief zu halten.

Eine bevorzugte Weiterbildung besteht darin, dass die nicht aus PTFE bestehende Materialschicht, vorzugsweise die äussere, dem Innenraum abgewandte Materialschicht, aus Polyesterfasern besteht. Die Partikelabgabe sollte weniger als 14'000, vorzugsweise weniger als 7'000 Partikel (der Grösse > 0,3 µm) pro Kubikmeter Luft und Minute betragen.

Die aus Polytetrafluorethylen bestehende Materialschicht ist vorteilhafterweise eine mikroporöse Membrane mit 0,4 bis 5 Milliarden, vorzugsweise 0,7 bis 3 Milliarden Poren pro cm² ist, um trotz der relativen Luftdichtheit eine gewisse Wasserdampfdurchlässigkeit zu gestatten. Die Porengrösse sollte 0,1 bis 0,4 µm, vorzugsweise 0,15 bis 0,25 µm betragen um die gewünschte Viren- und Keim-Dichtigkeit zu garantieren.

Die Dicke der aus Polytetrafluorethylen (PTFE). vorzugsweise e-Polytetrafluorethylen bestehenden Materialschicht sollte 0,01 bis 0,04 mm, vorzugsweise 0,015 bis 0,025 mm betragen.

Die luftdichte Oberflächenpartie der Wärmedecke ist vorzugsweise hydrophob und/oder flüssigkeitsdicht (minimal 700 mm Wassersäule). Die luftdurchlässige Oberflächenpartie und/oder die luftundurchlässige Oberflächenpartie ist antistatisch ausgebildet.

Um das zum Aufblasen der Wärmedecke benötigte Warmluftgebläse rasch und sicher anschliessen zu können, ist der Luftzufuhrstutzen zweckmässigerweise elastisch ausgebildet. Im weiteren hat es sich als vorteilhaft erwiesen den Luftzufuhrstutzen konstruktiv bis in den Innenraum hineinzuführen, so dass er ein Stück weit in diesen hineinragt. Die Luftverteilung im Innenraum wird dadurch wesentlich verbessert.

Zur sicheren Befestigung des Warmluftgebläses ist der Luftzufuhrstutzen mit Stoffbändeln oder anderen geeigneten Mitteln versehen, welche die Fixierung des Luftzufuhrstutzen an das Warmluftgebläse gestatten.
Bei einer bevorzugten Ausführungsform umfasst die luftdichte Oberflächenpartie drei übereinanderliegende Materialschichten, wobei die mittlere Materialschicht (7) aus e-Polytetrafluorethylen (PTFE) besteht.

Die Erfindung und Weiterbildungen der Erfindung werden im folgenden anhand der teilweise schematischen Darstellung eines Ausführungsbeispiels noch näher erläutert.
Es zeigen:
Fig. 1 einen Längsschnitt durch die erfindungsgemässe Vorrichtung;
Fig. 2 einen Längsschnitt durch die luftdichte Oberflächenpartie der Vorrichtung nach Fig. 1; und
Fig. 3 einen Längsschnitt durch eine Variante der luftdichten Oberflächenpartie der Vorrichtung nach Fig. 1.

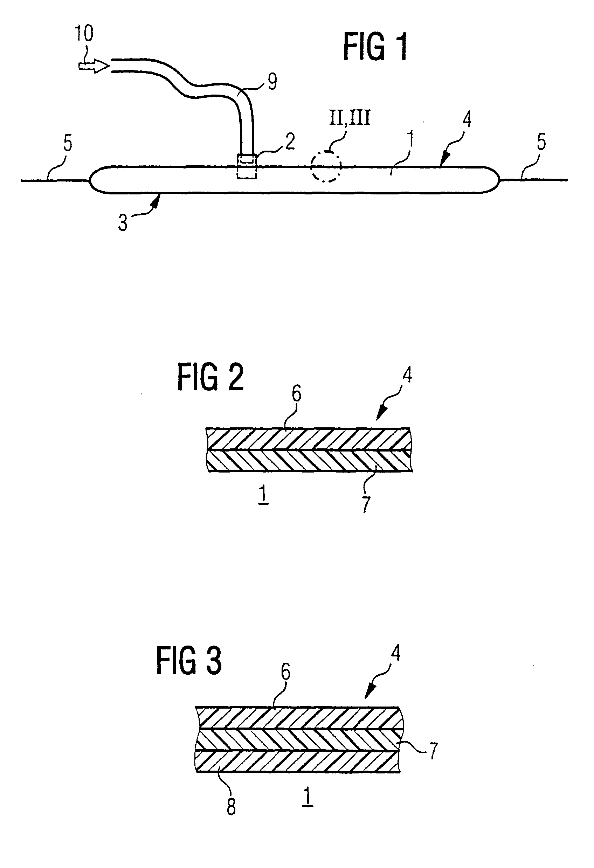

Die in Fig. 1 dargestellte Vorrichtung zum wärmeisolierenden Abdecken eines Patienten besteht im wesentlichen aus einer mit Luft aufblasbaren hohlen Wärmedecke.

Die Wärmedecke umfasst einen Innenraum 1, in welchen mittels eines Luftzufuhrstutzen 2 Warmluft eingeführt werden kann. Eine bekannte Warmluftquelle 10 kann mittels eines Schlauchs 9 an den - zu diesem Zweck elastisch und flexibel ausgebildeten - Luftzufuhrstutzen 2 angeschlossen und wieder leicht entfernt werden. Der Luftzufuhrstutzen 2 ragt ein Stück weit in den Innenraum 1 der Warmedecke hinein.

Die Wärmedecke weist eine luftdurchlässige Oberflächenpartie 3 und eine luft- und flüssigkeitsdichte Oberflächenpartie 4 auf, welche beide antistatisch ausgebildet sind. Die luftdurchlässige Oberflächenpartie 3 weist eine grössere Fläche als die luftdichte Oberflächenpartie 4 auf, so dass die luftdurchlässige Oberflächenpartie 3 für die hohle Wärmedecke einen peripher überstehenden Rand 5 bildet. Dieser Rand dient gleichzeitig als über den Operationstisch herausragende Abdeckung.

Bei einer bevorzugten - in Fig. 2 im Detail dargestellten - Ausführungsform umfasst die luftdichte Oberflächenpartie 4 zwei übereinanderliegende Materialschichten 6 und 7, wobei die eine der beiden Materialschichten 7, welche dem Innenraum 1 benachbart ist, aus e-Polytetrafluorethylen (PTFE) besteht.

Bei einer anderen Ausführungsform, welche in Fig. 3 im Detail dargestellt ist, umfasst die luftdichte Oberflächenpartie 4 drei übereinanderliegende Materialschichten 6,7,8, wobei die mittlere Materialschicht 7 aus e-Polytetrafluorethylen (PTFE) besteht.

Bei beiden Ausführungsformen (gemäss Fig. 2 oder 3) besteht, bzw. bestehen die anderen, nicht aus PTFE bestehenden Materialschichten (6;7) aus einem Gewebe von Polyester-Endlosfasern, welches weniger als 190 - 240 Partikel (mit einer Grösse > 0,3 µm) pro Kubikfuss Luft und pro Minute abgibt.

Die aus Polytetrafluorethylen (PTFE) bestehende Materialschicht (7;6) ist eine 0,02 mm dicke, mikroporöse Membrane mit 2 Milliarden, Poren pro cm², wobei die Porengrösse 0,2 µm ist.

Die erfindungsgemässe Vorrichtung kann entweder als herkömmliche, rechteckige oder quadratische Wärmedecke ausgebildet sein, welche mit der luftdurchlässigen Oberflächenpartie 3 prä-, intra- und postoperativ auf den Patienten gelegt werden kann um diesen vor Wärmeverlust zu schützen. Die Vorrichtung kann aber auch in jeder anderen beliebigen Form realisiert werden, z.B. als ein- oder zweiarmiger Schlauch (mit mittig angeordnetem Lufteintrittstutzen), welcher zum Beispiel um den Patienten herumgelegt wird, derart dass die luftdurchlässige Oberflächenpartie 3 gegen den Patienten gerichtet ist.

## Patentansprüche

1. Vorrichtung zum wärmeisolierenden Abdecken eines Patienten, welche eine mit Luft aufblasbare hohle Wärmedecke umfasst, die einen Innenraum (1), einen Luftzufuhrstutzen (2), eine luftdurchlässige Oberflächenpartie (3) und eine luftdichte Oberflächenpartie (4) aufweist, wobei
A) die luftdichte Oberflächenpartie (4) zwei übereinanderliegende Materialschichten (6,7) umfasst; und
B) eine der beiden Materialschichten (7;6) aus Polytetrafluorethylen (PTFE) besteht.
**dadurch gekennzeichnet, dass**
C) die andere der beiden Materialschichten (6;7) aus Endlosfasern besteht.

2. Vorrichtung nach Anspruch 1, **dadurch gekennzeichnet, dass** das Polytetrafluorethylen (PTFE) e-Polytetrafluorethylen ist.

3. Vorrichtung nach Anspruch 1 oder 2, **dadurch gekennzeichnet, dass** die andere der beiden Materialschichten (6;7), vorzugsweise die äussere, dem Innenraum (1) abgewandte Materialschicht (6), aus Polyesterfasern besteht.

4. Vorrichtung nach einem der Ansprüche 1 bis 3, **dadurch gekennzeichnet, dass** die andere der beiden Materialschichten (6;7) die äussere, dem Innenraum (1) abgewandte Materialschicht (6) ist.

5. Vorrichtung nach einem der Ansprüche 1 bis 4, **dadurch gekennzeichnet, dass** die aus Polytetrafluorethylen bestehende Materialschicht (7;6) eine mikroporöse Membrane mit 0,4 bis 5 Milliarden Poren pro cm² ist.

6. Vorrichtung nach Anspruch 5, **dadurch gekennzeichnet, dass** die aus Polytetrafluorethylen bestehende Materialschicht (7;6) eine mikroporöse Membrane mit 0,7 bis 3 Milliarden Poren pro cm² ist.

7. Vorrichtung nach Anspruch 5 oder 6, **dadurch gekennzeichnet, dass** die Porengrösse 0,1 bis 0,4 µm beträgt.

8. Vorrichtung nach Anspruch 7, **dadurch gekennzeichnet, dass** die Porengrösse 0,15 bis 0,25 µm beträgt.

9. Vorrichtung nach einem der Ansprüche 1 bis 8, **dadurch gekennzeichnet, dass** die aus Polytetrafluorethylen (PTFE) bestehende Materialschicht (7;6) 0,01 bis 0,04 mm dick ist.

10. Vorrichtung nach Anspruch 9, **dadurch gekennzeichnet, dass** die aus Polytetrafluorethylen (PTFE) bestehende Materialschicht (7;6) 0,015 bis 0,025 mm dick ist.

11. Vorrichtung nach einem der Ansprüche 1 bis 10, **dadurch gekennzeichnet, dass** die luftdichte Oberflächenpartie (4) hydrophob ist.

12. Vorrichtung nach einem der Ansprüche 1 bis 11, **dadurch gekennzeichnet, dass** die luftdichte Oberflächenpartie (4) flüssigkeitsdicht ist.

13. Vorrichtung nach einem der Ansprüche 1 bis 12, **dadurch gekennzeichnet, dass** die äussere, dem Innenraum (1) abgewandte Materialschicht (6) aus einem Gewebe besteht, welches weniger als 14'000 Partikel > 0,3 µm pro Kubikmeter Luft und pro Minute abgibt.

14. Vorrichtung nach Anspruch 13, **dadurch gekennzeichnet, dass** die äussere, dem Innenraum (1) abgewandte Materialschicht (6) aus einem Gewebe besteht, welches weniger als 7000 Partikel > 0,3 µm pro Kubikmeter Luft und pro Minute abgibt.

15. Vorrichtung nach einem der Ansprüche 1 bis 14, **dadurch gekennzeichnet, dass** die luftdurchlässige Oberflächenpartie (3) und/oder die luftundurchlässige Oberflächenpartie (4) antistatisch ausgebildet sind.

16. Vorrichtung nach einem der Ansprüche 1 bis 15, **dadurch gekennzeichnet, dass** der Luftzufuhrstutzen (2) elastisch ausgebildet ist.

17. Vorrichtung nach einem der Ansprüche 1 bis 16, **dadurch gekennzeichnet, dass** der Luftzufuhrstutzen (2) in den Innenraum (1) hineinragt.

18. Vorrichtung nach einem der Ansprüche 1 bis 17, **dadurch gekennzeichnet, dass** die luftdichte Oberflächenpartie (4) drei übereinanderliegende Materialschichten (6,7,8) umfasst, wobei die mittlere Materialschicht (7) aus Polytetrafluorethylen (PTFE), vorzugsweise aus e-Polytetrafluorethylen besteht.

19. Vorrichtung nach einem der Ansprüche 1 bis 18, **dadurch gekennzeichnet, dass** die luftdurchlässige Oberflächenpartie (3) eine grössere Fläche als die luftdichte Oberflächenpartie (4) aufweist.

20. Vorrichtung nach einem der Ansprüche 1 bis 19, **dadurch gekennzeichnet, dass** die luftdurchlässige Oberflächenpartie (3) für die hohle Wärmedecke einen peripher überstehenden Rand (5) bildet.

21. Vorrichtung nach einem der Ansprüche 1 bis 20, **dadurch gekennzeichnet, dass** die luftdurchlässige Oberflächenpartie (3) derart ausgebildet ist, dass sie die Unterseite der hohlen Wärmedecke bildet, welche auf oder seitlich zum Patienten zu liegen kommt.

## Claims

1. Device for heat-insulated covering of a patient comprising an air-inflatable hollow blanket, provided with an inner room (1), an air-inlet support (2), an air-permeable surface section (3) and an airtight surface section (4), wherein
A) the airtight surface section (4) comprises two material layers (6,7) lying on top of each other, and
B) one of the two material layers (6,7) is made of polytetrafluoroethlyene (PTFE),
**characterised in that**
C) the other of the two material layers (6,7) is made of endless fibres.

2. Device according to Claim 1, **characterised in that** the polytetrafluoroethlyene (PTFE) is e-polytetrafluoroethlyene.

3. Device according to Claim 1 or Claim 2, **characterised in that** the other of the two material layers (6,7), preferably the outer material layer (6) facing away from the inner room (1), is made of polyester fibres.

4. Device according to one of the claims 1 to 3, **characterised in that** the other of the two material layers (6,7) is the outer material layer (6) facing away from the inner room (1).

5. Device according to one of the claims 1 to 4, **characterised in that** the material layer (7,6) composed of polytetrafluoroethlyene is a micro-porous membrane with 0.4 to 5 billion pores per cm².

6. Device according to Claim 5, **characterised in that** the material layer (7,6) composed of polytetrafluoroethlyene is a micro-porous membrane with 0.7 to 3 billion pores per cm².

7. Device according to Claim 6 or Claim 6, **characterised in that** the pore size is 0.1 to 0.4 µm.

8. Device according to Claim 7, **characterised in that** the pore size is 0.15 to 0.25 µm.

9. Device according to one of the claims 1 to 8, **characterised in that** the material layer (7,6) composed of polytetrafluoroethlyene (PTFE) is 0.015 to 0.025 mm thick.

10. Device according to Claim 9, **characterised in that** the material layer (7,6) composed of polytetrafluoroethlyene (PTFE) is 0.01 to 0.04 mm thick.

11. Device according to one of the claims 1 to 10, **characterised in that** the airtight surface section (4) is of hydrophobic design.

12. Device according to one of the claims 1 to 11, **characterised in that** the airtight surface section (4) is watertight.

13. Device according to one of the claims 1 to 12, **characterised in that** the outer material layer (6) facing away from the inner room (1) consists of a tissue that emits less than 14,000 particles > 0.3 µm per cubic metre of air and per minute.

14. Device according to Claim 13, **characterised in that** the outer material layer (6) facing away from the inner room (1) consists of a tissue that emits less than 7,000 particles > 0.3 µm per cubic metre of air and per minute.

15. Device according to one of the claims 1 to 14, **characterised in that** the air-permeable surface section (3) and/or the airtight surface section (4) are anti-static.

16. Device according to one of the claims 1 to 15, **characterised in that** the air-inlet support (2) is elastic.

17. Device according to one of the claims 1 to 16, **characterised in that** the air-inlet support (2) protrudes into the inner room (1).

18. Device according to one of the claims 1 to 17, **characterised in that** the airtight surface section (4) comprises three material layers (6,7,8) lying on top of each other, wherein the middle material layer (7) consists of polytetrafluoroethlyene (PTFE) and preferably e-polytetrafluoroethlyene.

19. Device according to one of the claims 1 to 18, **characterised in that** the air-permeable surface section (3) has a greater area than the airtight surface section (4).

20. Device according to one of the claims 1 to 19, **characterised in that** the air-permeable surface section (3) forms a high peripheral rim (5) for the hollow covering.

21. Device according to one of the claims 1 to 20, **characterised in that** the air-permeable surface section (3) is designed in such a way that it forms the underside of the hollow covering which is laid on or at the side of the patient.

## Revendications

1. Dispositif pour couvrir un patient de façon thermo-isolante, comprenant une couverture de réchauffement creuse que l'on peut gonfler d'air et qui présente une chambre (1), un embout d'alimentation en air (2), une partie superficielle perméable à l'air (3) et une partie superficielle imperméable à l'air (4),
A) la partie superficielle imperméable à l'air (4) comprenant deux couches de matériau (6,7) superposées; et
B) une des deux couches de matériau (7;6) étant en polytétrafluoréthylène (PTFE),
**caractérisé en ce que**
C) l'autre des deux couches de matériau (6;7) est en filaments.

2. Dispositif selon la revendication 1, **caractérisé en ce que** le polytétrafluoréthylène (PTFE) est du polytétrafluoréthylène expansé.

3. Dispositif selon la revendication 1 ou 2, **caractérisé en ce que** l'autre des deux couches de matériau (6;7), de préférence la couche de matériau extérieure (6) située à l'opposé de la chambre (1), est en fibres de polyester.

4. Dispositif selon une des revendications 1 à 3, **caractérisé en ce que** l'autre des deux couches de matériau (6;7) est la couche de matériau extérieure (6) située à l'opposé de la chambre (1).

5. Dispositif selon une des revendications 1 à 4, **caractérisé en ce que** la couche de matériau en polytétrafluoréthylène (7;6) est une membrane microporeuse comptant 0,4 à 5 milliards de pores par cm².

6. Dispositif selon la revendication 5, **caractérisé en ce que** la couche de matériau en polytétrafluoréthylène (7;6) est une membrane microporeuse comptant 0,7 à 3 milliards de pores par cm².

7. Dispositif selon la revendication 5 ou 6, **caractérisé en ce que** la taille des pores est comprise entre 0,1 et 0,4 µm.

8. Dispositif selon la revendication 7, **caractérisé en ce que** la taille des pores est comprise entre 0,15 et 0,25 µm.

9. Dispositif selon une des revendications 1 à 8, **caractérisé en ce que** la couche de matériau en polytétrafluoréthylène (PTFE) (7;6) est épaisse de 0,01 à 0,04 mm.

10. Dispositif selon la revendication 9, **caractérisé en ce que** la couche de matériau en polytétrafluoréthylène (PTFE) (7;6) est épaisse de 0,015 à 0,025 mm.

11. Dispositif selon une des revendications 1 à 10, **caractérisé en ce que** la partie superficielle imperméable à l'air (4) est hydrophobe.

12. Dispositif selon une des revendications 1 à 11, **caractérisé en ce que** la partie superficielle imperméable à l'air (4) est imperméable aux liquides.

13. Dispositif selon une des revendications 1 à 12, **caractérisé en ce que** la couche de matériau extérieure (6) située à l'opposé de la chambre (1) est faite d'un tissu qui libère moins de 14000 particules > 0,3 µm par mètre cube d'air et par minute.

14. Dispositif selon la revendication 13, **caractérisé en ce que** la couche de matériau extérieure (6) située à l'opposé de la chambre (1) est faite d'un tissu qui libère moins de 7000 particules > 0,3 µm par mètre cube d'air et par minute.

15. Dispositif selon une des revendications 1 à 14, **caractérisé en ce que** la partie superficielle perméable à l'air (3) et/ou la partie superficielle imperméable à l'air (4) sont antistatiques.

16. Dispositif selon une des revendications 1 à 15, **caractérisé en ce que** l'embout d'alimentation en air (2) est élastique.

17. Dispositif selon une des revendications 1 à 16, **caractérisé en ce que** l'embout d'alimentation en air (2) pénètre dans la chambre (1).

18. Dispositif selon une des revendications 1 à 17, **caractérisé en ce que** la partie superficielle imperméable à l'air (4) comprend trois couches de matériaux (6,7,8) superposées, la couche de matériau du milieu (7) étant en polytétrafluoréthylène (PTFE), de préférence en polytétrafluoréthylène expansé.

19. Dispositif selon une des revendications 1 à 18, **caractérisé en ce que** la partie superficielle perméable à l'air (3) présente une surface plus grande que celle de la partie superficielle imperméable à l'air (4).

20. Dispositif selon une des revendications 1 à 19, **caractérisé en ce que** la partie superficielle perméable à l'air (3) forme une bordure (5) dépassant sur le pourtour pour la couverture de réchauffement creuse.

21. Dispositif selon une des revendications 1 à 20, **caractérisé en ce que** la partie superficielle perméable à l'air (3) est conçue de façon à former la face inférieure de la couverture de réchauffement creuse que l'on place sur ou le long du patient.
